# EUROPEAN PATENT APPLICATION

(11) **EP 4 169 521 A1**
(43) Date of publication of application: **26.04.2023**
(21) Application number: 21826238.4
(22) Date of filing: 21.04.2021
(51) Int. Cl.: A61K 35/745, A61P 25/28

(54) **COGNITIVE FUNCTION IMPROVING AGENT, COGNITIVE FUNCTION MAINTAINING AGENT, HIPPOCAMPUS FUNCTION IMPROVING AGENT, AND HIPPOCAMPUS FUNCTION MAINTAINING AGENT**

(30) Priority: 18.06.2020 JP 2020105650
(71) Applicant: Morinaga Milk Industry Co., Ltd., Minato-ku Tokyo 108-8384 (JP)
(72) Inventor: SHIMIZU Kanetada, Zama-shi, Kanagawa 252-8583 (JP); KATSUMATA Noriko, Zama-shi, Kanagawa 252-8583 (JP); ONO Kazuya, Zama-shi, Kanagawa 252-8583 (JP)
(74) Representative: Hoffmann Eitle
(86) International application number: PCT/JP2021/016150
(87) International publication number: WO 2021/256077

(57) **Abstract**

A cognitive function-improving agent containing one or both of *Bifidobacterium breve* FERM BP-11175 and a culture of *Bifidobacterium breve* FERM BP-11175 as an active ingredient for administration to a subject who has not developed dementia is provided.

## Description

### Technical Field

The present invention relates to a cognitive function-improving agent, a cognitive function-maintaining agent, a hippocampus function-improving agent and a hippocampus function-maintaining agent.

The present application claims priority from patent application No. 2020-105650 filed on June 18, 2020 in Japan, and the contents thereof are incorporated here by reference.

### Background Art

Brain functions of humans, such as learning ability and memory, gradually decline with age, and forgetfulness, anxiety disorder, decrease in motivation, decrease in sleep quality and the like are apt to be caused. In addition to the laws of nature, when a person is afflicted with dementia, a serious situation which may even threaten human dignity may be caused. At the moment, prevention and treatment of dementia are energetically studied.

Dementia refers to a condition in which the patient continuously has problems in the life due to the inability to make decisions and rapid decline in memory and which is caused by various disorders caused by brain cell death due to various causes or deterioration of the functions. The major factor for developing dementia is neurodegenerative diseases, and the next factor is cerebrovascular dementia. In neurodegenerative diseases, abnormalities accumulate in neurons in the brain over a long time, which leads to cell death in the end, and Alzheimer's disease, frontotemporal dementia, Lewy body dementia and the like are included. Cerebrovascular dementia is caused by death of neurons in the brain or damage to the neural networks triggered by cerebral infarction, brain hemorrhage, cerebral arteriosclerosis or the like.

In PTL 1, a brain function-improving agent for a dementia patient is described.

### Citation List

### Patent Literature

PTL 1: WO2017/209156

### Summary of Invention

### Technical Problem

Mild cognitive impairment (MCI) is a condition in which the individual is healthy but in which decline in cognitive function is caused. MCI is a reversible condition, and the cognitive function can recover to the normal condition. Accordingly, even after a diagnosis with MCI, transition to dementia may be delayed or prevented when the cognitive function can be improved or maintained.

Alzheimer-type dementia accounts for nearly a half of the dementia cases. It is considered that, in Alzheimer-type dementia, degeneration of neurons is observed 20 years before the onset or before that and that decline in hippocampus function is caused by falling of neurons and hippocampal atrophy. Accordingly, the onset of Alzheimer-type dementia may be delayed or prevented when the hippocampus function can be improved or maintained.

An object of the invention is to provide a cognitive function-improving agent, a cognitive function-maintaining agent, a hippocampus function-improving agent or a hippocampus function-maintaining agent for administration to a subject who has not developed dementia.

### Solution to Problem

[1] A cognitive function-improving agent containing one or both of *Bifidobacterium breve* FERM BP-11175 and a culture of *Bifidobacterium breve* FERM BP-11175 as an active ingredient for administration to a subject who has not developed dementia.
[2] The cognitive function-improving agent described in [1], wherein the subject who has not developed dementia is a subject who has not developed dementia but who is concerned about age-related decline in cognitive function of the brain.
[3] The cognitive function-improving agent described in [1], wherein the subject who has not developed dementia is a subject who has not developed dementia but who is concerned about age-related decline in combined cognitive function.
[4] The cognitive function-improving agent described in [1], wherein the subject who has not developed dementia is selected from the group consisting of healthy individuals, the group consisting of individuals with mild cognitive impairment and the group consisting of healthy individuals and individuals with mild cognitive impairment.
[5] The cognitive function-improving agent described in [1], wherein the subject who has not developed dementia is a subject having an MMSE score of 22 or more and having a total RBANS score of 65 or less or a Japanese-version of the MCI Screen score of 75 or less.
[6] The cognitive function-improving agent described in any of [1] to [5] which contains 1×10⁶ to 1×10¹² CFU/g of *Bifidobacterium breve.*
[7] A cognitive function-improving food or drink containing one or both of *Bifidobacterium breve* FERM BP-11175 and a culture of *Bifidobacterium breve* FERM BP-11175 for a subject who has not developed dementia.
[8] Use of one or both of *Bifidobacterium breve* FERM BP-11175 and a culture of *Bifidobacterium breve* FERM BP-11175 for the manufacture of a cognitive function-improving agent for administration to a subject who has not developed dementia.
[9] A cognitive function-maintaining agent containing one or both of *Bifidobacterium breve* FERM BP-11175 and a culture of *Bifidobacterium breve* FERM BP-11175 as an active ingredient for administration to a subject who has not developed dementia.
[10] The cognitive function-maintaining agent described in [9], wherein the subject who has not developed dementia is a subject who has not developed dementia but who is concerned about age-related decline in cognitive function of the brain.
[11] The cognitive function-maintaining agent described in [9], wherein the subject who has not developed dementia is a subject who has not developed dementia but who is concerned about age-related decline in combined cognitive function.
[12] The cognitive function-maintaining agent described in [9], wherein the subject who has not developed dementia is selected from the group consisting of healthy individuals, the group consisting of individuals with mild cognitive impairment and the group consisting of healthy individuals and individuals with mild cognitive impairment.
[13] The cognitive function-maintaining agent described in [9], wherein the subject who has not developed dementia is a subject having an MMSE score of 22 or more and having a total RBANS score of 65 or less or a Japanese-version of the MCI Screen score of 75 or less.
[14] The cognitive function-maintaining agent described in any of [9] to [13] which contains 1×10⁶ to 1×10¹² CFU/g of *Bifidobacterium breve.*
[15] A cognitive function-maintaining food or drink containing one or both of *Bifidobacterium breve* FERM BP-11175 and a culture of *Bifidobacterium breve* FERM BP-11175 for a subject who has not developed dementia.
[16] Use of one or both of *Bifidobacterium breve* FERM BP-11175 and a culture of *Bifidobacterium breve* FERM BP-11175 for the manufacture of a cognitive function-maintaining agent for administration to a subject who has not developed dementia.
[17] A hippocampus function-improving agent containing one or both of *Bifidobacterium breve* FERM BP-11175 and a culture of *Bifidobacterium breve* FERM BP-11175 as an active ingredient.
[18] The hippocampus function-improving agent described in [17] for administration to a subject who has not developed dementia.
[19] The hippocampus function-improving agent described in [18], wherein the subject who has not developed dementia is selected from the group consisting of healthy individuals, the group consisting of individuals with mild cognitive impairment and the group consisting of healthy individuals and individuals with mild cognitive impairment.
[20] The hippocampus function-improving agent described in [18], wherein the subject who has not developed dementia is a subject having an MMSE score of 22 or more and having a total RBANS score of 65 or less or a Japanese-version of the MCI Screen score of 75 or less.
[21] The hippocampus function-improving agent described in any of [17] to [20] which contains 1×10⁶ to 1×10¹² CFU/g of *Bifidobacterium breve.*
[22] A hippocampus function-improving food or drink containing one or both of *Bifidobacterium breve* FERM BP-11175 and a culture of *Bifidobacterium breve* FERM BP-11175.
[23] Use of one or both of *Bifidobacterium breve* FERM BP-11175 and a culture of *Bifidobacterium breve* FERM BP-11175 for the manufacture of a hippocampus function-improving agent.
[24] A hippocampus function-maintaining agent containing one or both of *Bifidobacterium breve* FERM BP-11175 and a culture of *Bifidobacterium breve* FERM BP-11175 as an active ingredient.
[25] The hippocampus function-maintaining agent described in [24] for administration to a subject who has not developed dementia.
[26] The hippocampus function-maintaining agent described in [25], wherein the subject who has not developed dementia is selected from the group consisting of healthy individuals and individuals with mild cognitive impairment.
[27] The hippocampus function-maintaining agent described in [25], wherein the subject who has not developed dementia is a subject having an MMSE score of 22 or more and having a total RBANS score of 65 or less or a Japanese-version of the MCI Screen score of 75 or less.
[28] The hippocampus function-maintaining agent described in any of [24] to [27] which contains 1×10⁶ to 1×10¹² CFU/g of *Bifidobacterium breve.*
[29] A hippocampus function-maintaining food or drink containing one or both of *Bifidobacterium breve* FERM BP-11175 and a culture of *Bifidobacterium breve* FERM BP-11175.
[30] Use of one or both of *Bifidobacterium breve* FERM BP-11175 and a culture of *Bifidobacterium breve* FERM BP-11175 for the manufacture of a hippocampus function-maintaining agent.

### Advantageous Effects of Invention

According to the invention, a cognitive function-improving agent, a cognitive function-maintaining agent, a hippocampus function-improving agent or a hippocampus function-maintaining agent for administration to a subject who has not developed dementia can be provided.

### Brief Description of Drawings

[Fig. 1] Fig. 1 is a graph showing the RBANS scores before and after the trial.
[Fig. 2] Fig. 2 is a graph showing the changes in the RBANS scores before and after the trial.
[Fig. 3] Fig. 3 is a graph showing the scores and the changes in the Japanese-version of the MCI Screen (JMCIS) before and after the trial.

### Description of Embodiments

A numerical range expressed with "to" includes the values before and after the "to".

An MMSE score is a score calculated by the MMSE (Mini Mental State Examination) . The MMSE is a screening method for cognitive dysfunctions which is widely used internationally for clinical sites and studies. This is an examination regarding orientation to time and place, immediate recall, short-term verbal memory, calculation, language, and construct ability, and the maximum score is 30 points . A lower MMSE score indicates that the decline in cognitive function is advanced.

An RBANS score is a score calculated by the RBANS (Repeatable Battery for the Assessment of Neuropsychological Status) . The RBANS was developed in 1998 by Randolph, and five cognition domains of immediate memory, delayed memory, visuospatial/constructional, language and attention are assessed with the neuropsychological assessment standardized in the U.S. The assessment consists of twelve tests, and the assessment score of each cognition domain is calculated from the approximate score of each test based on the conversion table considering the age and the like (provided by Resilio Co ., Ltd.). A lower RBANS score indicates that the decline in cognitive function is advanced.

The Japanese-version of the MCI Screen (JMCIS) (registered trademark) is an examination which can quantitatively express a minute change in cognitive function in the healthy to border to MCI regions easily with high accuracy by interactive checking for around 10 minutes. Based on the information on the subject, such as the gender, the age, the learning period (years) and the race, the memory performance index is calculated using the unique algorithm and database (provided by MILLENNIA Corporation) . The MPI (Memory Performance Index) score (called "MPI score" or "Japanese-version of the MCI Screen score" below) is an index objectively indicating the cognitive function of an individual assessed and calculated by the Japanese-version of the MCI Screen, and a lower MPI score indicates that the decline in cognitive function is advanced.

FERM is a symbol indicating the NITE International Patent Organism Depositary (NITE-IPOD) . NITE is the abbreviation for the National Institute of Technology and Evaluation.

### [Cognitive Function-Improving Agent]

The cognitive function-improving agent of the invention is a cognitive function-improving agent containing one or both of *Bifidobacterium breve* FERM BP-11175 and a culture of *Bifidobacterium breve* FERM BP-11175 as an active ingredient.

### <Subject of Administration>

The subject of administration of the cognitive function-improving agent of the invention is a subject who has not developed dementia.

The subject is generally human, but administration to a mammal other than human, such as pet animals including dog, cat and the like and livestock including cow, sheep, pig and the like, is also possible. The cases in which the subject is human are explained below.

The subject who has not developed dementia is preferably any subject of (1) to (4) below.
(1) A subject who has not developed dementia but who is concerned about age-related decline in cognitive function of the brain.
(2) A subject who has not developed dementia but who is concerned about age-related decline in combined cognitive function.
(3) A subject selected from the group consisting of healthy individuals, the group consisting of individuals with mild cognitive impairment and the group consisting of healthy individuals and individuals with mild cognitive impairment.
(4) A subject having an MMSE score of 22 or more and having a total RBANS score of 65 or less or a Japanese-version of the MCI Screen score of 75 or less. The total RBANS score is preferably 41 or less. The Japanese-version of the MCI Screen score is preferably 50.2 or less.

### <Administration Route>

Examples of the administration route of the cognitive function-improving agent of the invention include oral administration, intraperitoneal administration, intramuscular administration, transmucosal administration, intranasal administration, rectal administration and the like, but oral administration is particularly preferable.

### <Improvement of Cognitive Function>

In the invention, the improvement of cognitive function means that an increase in score is observed in one or more of the MMSE score, the total RBANS score and the Japanese-version of the MCI Screen before and after the administration of the cognitive function-improving agent of the invention. In particular, as the improvement of cognitive function, it is preferable that an increase in score is observed in one or more of the domains of immediate memory, visuospatial/constructional and delayed memory and the total score in the RBANS, and it is more preferable that an increase in score is observed in at least one of the domains of visuospatial/constructional and delayed memory. Moreover, it is desirable that an increase in the MPI score in the Japanese-version of the MCI Screen is observed.

### <Active Ingredient>

The active ingredient of the cognitive function-improving agent of the invention is one or both of *Bifidobacterium breve* FERM BP-11175 and a culture of *Bifidobacterium breve* FERM BP-11175.

One or both of *Bifidobacterium breve* FERM BP-11175 and a culture of *Bifidobacterium breve* FERM BP-11175 are used for the manufacture of the cognitive function-improving agent of the invention.

*Bifidobacterium breve* is a species of bacteria belonging to *Bifidobacterium. Bifidobacterium breve* mainly lives in the large intestine of infants and small children in a high amount and is known as an infant- and small children-type *Bifidobacterium* species together with *Bifidobacterium longum* subsp. *infantis* and the like of the bacterial species belonging to *Bifidobacterium.*

The cognitive function-improving agent of the present technology is highly safe, is not likely to cause need for worries for side effects even after long-term continuous administration and is very useful because the active ingredient is one or both of *Bifidobacterium breve,* which mainly lives in the large intestine of infants and small children in a high amount, and a culture containing *Bifidobacterium breve.* Moreover, the safety is high even in a combination use with another drug.

Instead of *Bifidobacterium breve* FERM BP-11175, an equivalent bacterial strain may also be used. The equivalent bacterial strain means a bacterial strain derived from the identical isolate. An example of an equivalent bacterial strain of *Bifidobacterium breve* FERM BP-11175 is *Bifidobacterium breve* MCC1274. Here, MCC means Morinaga Culture Collection (Morinaga Milk Industry Co., Ltd.).

*Bifidobacterium breve* FERM BP-11175 can be easily obtained by culturing the furnished strain of *Bifidobacterium breve* FERM BP-11175. The culture method is not particularly limited, and the bacterium can be cultured under appropriate conditions according to the properties of the bacterium.

Specifically, for example, the culture temperature is generally 25 to 50°C, preferably 35 to 42°C. The bacterium is preferably cultured under an anaerobic condition and can be cultured, for example, while sending an anaerobic gas such as carbon dioxide gas. Furthermore, the bacterium may be cultured under a microaerophilic condition such as static liquid culture.

The medium for culturing *Bifidobacterium breve* FERM BP-11175 is not particularly limited, and a medium which is generally used for culturing a bacterium of *Bifidobacterium* can be used.

That is, as a carbon source, for example, saccharides such as glucose, galactose, lactose, arabinose, mannose, sucrose, starch, starch hydrolysate and molasses can be used depending on the assimilation properties. As a nitrogen source, for example, ammonia and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, ferrous sulfate and the like can be used. Furthermore, organic components such as peptone, soybean powder, a defatted soybean cake, meat extract and yeast extract may also be used.

As *Bifidobacterium breve* FERM BP-11175, the obtained culture may be used directly after culture or used after dilution or concentration, or bacterial cells collected from the culture may also be used.

For the cognitive function-improving agent of the invention, not only *Bifidobacterium breve* FERM BP-11175 but also a culture or a culture supernatant containing *Bifidobacterium breve* FERM BP-11175 can be used as the active ingredient.

As the culture, a culture obtained by culturing and growing *Bifidobacterium breve* FERM BP-11175 and then removing components other than the bacterial cells as much as possible is preferable, and a culture consisting substantially of bacterial cells is more preferable. Here, "consisting substantially of bacterial cells" means that 95 mass% or more of the total mass of the culture, by wet mass, is bacterial cells.

The culture supernatant is the culture solution part obtained by culturing *Bifidobacterium breve* FERM BP-11175 or a supernatant solution thereof, and the culture supernatant is preferably sterilized by filtering.

The cognitive function-improving agent of the invention may consist of the active ingredient or may be a composition obtained by blending the active ingredient and an optional component other than the active ingredient.

The optional component is not particularly limited, and an additive which has been blended in a pharmaceutical product (for example, the carriers for formulation described below and the like) can be blended.

The *Bifidobacterium breve* FERM BP-11175 content of the cognitive function-improving agent of the invention is not particularly restricted, but *Bifidobacterium breve* is preferably contained in an amount which allows reasonable consumption of a daily dosage for effective cognitive function-improving effect and is more preferably contained in an amount of 1×10⁶ to 1×10¹² CFU/g.

Moreover, it is preferable to administer 1 to 3 g per day of the cognitive function-improving agent of the invention containing 1×10⁶ to 1×10¹² CFU/g of *Bifidobacterium breve* FERM BP-11175. In this regard, CFU indicates colony forming unit.

The cognitive function-improving agent of the invention containing 1×10⁶ to 1×10¹² CFU/g of *Bifidobacterium breve* FERM BP-11175 may be administered once a day but is preferably administered in two or more divided portions per day.

The cognitive function-improving agent of the invention containing 1×10⁶ to 1×10¹² CFU/g of *Bifidobacterium breve* FERM BP-11175 is preferably administered every day continuously and is more preferably administered every day, for example, for 12 weeks or longer.

### <Formulation>

The cognitive function-improving agent of the invention can be appropriately formulated into a desired dosage form depending on the administration method such as oral administration and parenteral administration. The dosage form is not particularly limited, but in the case of oral administration, for example, the cognitive function-improving agent can be formulated into a solid preparation such as powder, granules, tablets, troches and capsules, a liquid preparation such as a solution, a syrup, a suspension and an emulsion or the like. In the case of parenteral administration, for example, the cognitive function-improving agent can be formulated into a suppository, spray, inhalant, ointment, a plaster, an injection or the like. In the present technology, formulation into a dosage form for oral administration is preferable.

The formulation can be appropriately conducted by a known method depending on the dosage form.

The formulation may also be conducted, for example, by appropriately blending a carrier for formulation. Moreover, in addition to the cognitive function-improving agent of the invention, a component which is generally used for formulation, such as excipients, pH-adjusting agents, colorants and corrigents, can be used. A component having the effect of preventing, treating and/or improving a disease or a symptom which is known or will be found in the future can also be appropriately used in combination depending on the purpose.

As the carrier for formulation, an organic or inorganic carrier can be used depending on the dosage form.

Examples of the carrier for a solid preparation include excipients, binders, disintegrating agents, lubricants, stabilizers, flavoring agents and the like.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of the binders include, in addition to the excipients, gelatin, polyvinylpyrrolidone, macrogol and the like.

Examples of the disintegrating agents include, in addition to the excipients, chemically modified starch or cellulose derivatives such as croscarmellose sodium, sodium carboxymethyl starch and cross-linked polyvinylpyrrolidone and the like.

Examples of the lubricants include: talc; stearic acid; metal stearates such as calcium stearate and magnesium stearate; colloidal silica; waxes such as veegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of the flavoring agents include sweeteners, acidulants, aromas and the like.

In this regard, the carrier used in the case of a liquid preparation for oral administration is a solvent such as water, a flavoring agent or the like.

The cognitive function-improving agent of the invention can also be used in combination with a drug having cognitive function-improving effect, a drug having anti-dementia effect, a drug having anti-depression effect, a drug having anti-schizophrenic effect, a drug having anti-delirium effect or the like which is known or will be found in the future.

### [Cognitive Function-Improving Food or Drink]

The cognitive function-improving food or drink of the invention is a cognitive function-improving food or drink for a subject who has not developed dementia and contains one or both of *Bifidobacterium breve* FERM BP-11175 and a culture of *Bifidobacterium breve* FERM BP-11175.

The subject who has not developed dementia, *Bifidobacterium breve* FERM BP-11175 and the culture of *Bifidobacterium breve* FERM BP-11175 as the active ingredient are the same as those of the cognitive function-improving agent of the invention described above.

The food or the drink is not limited regarding the form such as liquid, paste, solid and powder, and examples thereof include, in addition to tablet candies, liquid foods, feed (including food for pets) and the like, wheat products, instant foods, processed agricultural products, processed fishery products, processed livestock products, milk-dairy products, oils and fats, basic condiments, compound flavor enhancers·foods, frozen foods, confectioneries, drinks, other commercial foods and the like.

Examples of the dairy products include fermented milk, milk beverages, lactic acid bacteria beverages, sweetened condensed milk, skim milk powder, sweetened milk powder, powdered formula, creams, cheeses, butter, ice creams and the like.

Examples of the wheat products include breads, macaroni, spaghetti, noodles, cake mixes, frying flours, bread crumbs and the like.

Examples of the instant foods include instant noodles, cup noodles, retort-pouched-prepared foods, prepared canned foods, microwave foods, instant soups-stews, instant miso soups-clear Japanese soups, canned soups, freeze-dried foods, other instant foods and the like.

Examples of the processed agricultural products include canned agricultural products, canned fruits, jams·marmalades, pickles, cooked beans, dried agricultural products, cereals (processed grains) and the like.

Examples of the processed fishery products include canned fishery products, fish hams-sausages, fishery paste products, fishery delicacies, *Tsukudani* (foods boiled down in sweetened soy sauce) and the like.

Examples of the processed livestock products include canned livestock products-pastes, livestock hams-sausages and the like.

Examples of the oils and fats include butter, margarine, vegetable oils and the like.

Examples of the basic condiments include soy sauce, soybean paste, sauces, processed tomato condiments, *Mirin* (sweet sake for seasoning), vinegars and the like, and the compound flavor enhancers·foods include cooking mixes, curry roux, sauces, dressings, noodle broths, spices, other compound flavor enhancers and the like.

Examples of the frozen foods include frozen food materials, semi-cooked frozen foods, cooked frozen foods and the like.

Examples of the confectioneries include caramels, candies, chewing gums, chocolates, cookies, biscuits, cakes, pies, snacks, crackers, Japanese-style confectioneries, rice confectioneries, bean confectioneries, desserts, other confectioneries and the like.

Examples of the drinks include carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols, other luxury beverages and the like.

Examples of the other commercial foods include baby foods, *Furikake* (dry Japanese seasonings), seasonings for *Chazuke* (boiled rice with hot tea) and the like.

Of these, the cognitive function-improving food or drink of the invention is particularly preferably a dairy product, particularly preferably fermented milk. As a result, in addition to the cognitive function-improving effect, the high nutritional value of a dairy product can also be enjoyed.

The cognitive function-improving food or drink of the invention can be applied to foods with function claims labeled with cognitive function-improving effect.

### [Cognitive Function-Maintaining Agent]

The cognitive function-maintaining agent of the invention is a cognitive function-maintaining agent containing one or both of *Bifidobacterium breve* FERM BP-11175 and a culture of *Bifidobacterium breve* FERM BP-11175 as an active ingredient.

The subject of administration, the administration route, the active ingredient and the formulation of the cognitive function-maintaining agent of the invention are the same as those of the cognitive function-improving agent of the invention described above.

### <Maintenance of Cognitive Function>

In the invention, the maintenance of cognitive function means that no significant decrease in score is observed in one or more of the MMSE score, the total RBANS score and the Japanese-version of the MCI Screen before and after the administration of the cognitive function-maintaining agent of the invention. In particular, as the maintenance of cognitive function, it is preferable that no significant decrease in score is observed in one or more of the domains of immediate memory, visuospatial/constructional and delayed memory and the total score in the RBANS, and it is more preferable that no significant decrease in score is observed in at least one of the domains of visuospatial/constructional and delayed memory. Moreover, it is desirable that no significant decrease in the MPI score is observed in the Japanese-version of the MCI Screen.

### [Cognitive Function-Maintaining Food or Drink]

The cognitive function-maintaining food or drink is the same as the cognitive function-improving food or drink of the invention described above.

The cognitive function-maintaining food or drink of the invention can be applied to foods with function claims labeled with cognitive function-maintaining effect.

### [Food or Drink with Functional Label]

The food, the drink or the like defined in the present technology can also be provided·sold as a food or a drink with a label with a specific use (especially a health use) or a function.

The "labeling" act includes all the acts for informing a consumer of the use, and all the expressions which can remind of-cause to guess the use are the "labeling" acts of the present technology, regardless of the purposes of labeling, the contents of labeling, the objects to be labeled, the media and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and another act.

The content of the label is preferably a label approved by the administration or the like (for example, a label approved based on a system provided by the administration and provided in the form based on the approval or the like) . It is preferable to label with such a content on packaging, a container, a catalogue, a brochure, an advertisement material in a sales site such as POP, other documents or the like.

The "labels" also include labels with health foods, functional foods, foods for the ill, enteral nutrition products, food for special dietary uses, food with health claims, foods for specified health uses, foods with function claims, foods with nutrient function claims, quasi-drugs and the like. In particular, the labels are labels approved by the Consumer Affairs Agency, such as labels approved by the systems for foods for specified health uses, the systems for foods with function claims and similar systems thereof. More specific examples include a label with foods for specified health uses, a label with qualified foods for specified health uses, a label with foods with function claims, a label indicating influence on the structure and the function of a body, a label with reduction of disease risk and the like. Typical examples thereof include labels with food for specified health uses (especially labels with health uses) provided by the Regulations for Enforcement of the Health Promotion Act (Order of the Ministry of Health, Labour and Welfare of Japan, No. 86, April 30, 2003), labels with foods with function claims provided by the Food Labeling Act (Act No. 70 of 2013) and similar labels.

The terms used for the labeling described above are not limited only to the terms such as enhancement-improvement-prevention of cognitive function, and other terms are of course included in the scope of the present technology as long as the terms indicate the effect of preventing, treating and/or improving a disease or a symptom related to cognition or learning. The terms can be, for example, labels based on various uses which allow a consumer to recognize the effect of maintaining-promoting cognitive·learning function or improving the accuracy, such as "for those who are concerned about decline in memory", "for those who wish to enhance cognitive function", "for those who are concerned about cognitive function", "for those who wish to prevent developing Alzheimer-type dementia in the future", "for those who wish to enhance cognitive accuracy", "for those who wish to support information of numbers, words, shapes, situation and the like", "for those who wish to enhance accuracy of memory or correctness of judgement as a part of cognitive function", "for those who wish to maintain memory", "for healthy middle-aged and elderly who wish to support maintenance of cognitive function", "for those who are concerned about forgetfulness and mild cognitive impairment (MCI)", "for the middle-aged and elderly who are concerned about forgetfulness" and "for the middle-aged and elderly who are concerned about mild cognitive impairment (MCI)".

### [Hippocampus Function-Improving Agent]

The hippocampus function-improving agent of the invention is a hippocampus function-improving agent containing one or both of *Bifidobacterium breve* FERM BP-11175 and a culture of *Bifidobacterium breve* FERM BP-11175 as an active ingredient.

The hippocampus is a part of the hippocampal formation as a part of the limbic system and is involved in memory and spatial learning ability of the brain. The hippocampus is known as the major part of lesions in Alzheimer-type dementia. Improvement of hippocampus function has significance particularly in preventing or delaying the onset of Alzheimer-type dementia.

The subject of administration of the hippocampus function-improving agent of the invention is not particularly limited but is preferably a subject who has not developed dementia.

The subject who has not developed dementia is the same as the subject of administration of the cognitive function-improving agent of the invention described above.

The administration route, the active ingredient and the formulation of the hippocampus function-improving agent of the invention are the same as those of the cognitive function-improving agent of the invention described above.

### <Improvement of Hippocampus Function>

In the invention, as the improvement of hippocampus function, it is preferable that an increase in score is observed in one or more of the domains of immediate memory, visuospatial/constructional and delayed memory governed by the hippocampus and the total score in the RBANS before and after the administration of the hippocampus function-improving agent of the invention, and it is preferable that an increase in score is observed in at least one of the domains of visuospatial/constructional and delayed memory.

### [Hippocampus Function-Improving Food or Drink]

The hippocampus function-improving food or drink is the same as the cognitive function-improving food or drink of the invention described above.

The hippocampus function-improving food or drink of the invention can be applied to foods with function claims labeled with hippocampus function-improving effect.

### [Hippocampus Function-Maintaining Agent]

The hippocampus function-maintaining agent of the invention is a hippocampus function-improving agent containing one or both of *Bifidobacterium breve* FERM BP-11175 and a culture of *Bifidobacterium breve* FERM BP-11175 as an active ingredient.

The subject of administration, the administration route, the active ingredient and the formulation of the hippocampus function-maintaining agent of the invention are the same as those of the hippocampus function-improving agent of the invention described above.

### <Maintenance of Hippocampus Function>

In the invention, as the maintenance of hippocampus function, it is preferable that no significant decrease in score is observed in one or more of the domains of immediate memory, visuospatial/constructional and delayed memory governed by the hippocampus and the score in the RBANS before and after the administration of the hippocampus function-improving agent of the invention, and it is more preferable that no significant decrease in score is observed in at least one of the domains of visuospatial/constructional and delayed memory.

### [Hippocampus Function-Maintaining Food or Drink]

The hippocampus function-maintaining food or drink is the same as the cognitive function-improving food or drink of the invention described above.

The hippocampus function-maintaining food or drink of the invention can be applied to foods with function claims labeled with hippocampus function-maintaining effect.

### Examples

The invention is described in detail below using Examples . In this regard, the Examples show typical examples of the invention, and the scope of the invention is not construed as being narrower due to the Examples.

### Trial Example of Higher Brain Function-Improving Effect of Probiotic Consumption in Subjects with Suspected Mild Cognitive Impairment - Placebo-Controlled, Randomized, Double-Blind, Parallel-Group Trial

Males and females aged 50 years or older and younger than 80 years with Japanese nationality were gathered from a volunteer bank, and 80 individuals were chosen from those who satisfied the inclusion criteria and who did not fall in the exclusion criteria based on the MMSE and RBANS scores.

The inclusion criteria of the participants were the following three points.
(1) Male or female aged 50 years or older and younger than 80 years with Japanese nationality.
(2) Subject having an MMSE score of 22 or more.
(3) Subject who has given the informed consent.

The exclusion criteria of the participants were the following 17 points.
(1) Subject diagnosed with dementia.
(2) Subject who is currently under exercise or dietetic therapy under the supervision of a doctor.
(3) Subject who may develop an allergy to the test food.
(4) Subject with a present or previous illness of drug dependence or alcohol dependence.
(5) Subject who regularly visits a doctor due to a psychiatric disorder (depression or the like), sleep disorder or the like or who is with a previous illness of a psychiatric disorder.
(6) Subject who works at night or has shift work.
(7) Subject whose lifestyle, such as diets and sleep, is extremely irregular.
(8) Subject who has an extremely unbalanced diet.
(9) Subject with a present or previous illness or a complication of a severe disease such as diabetes, liver disease (hepatitis), kidney disease and heart disease, thyroid disease, adrenal disease or another severe metabolic disease.
(10) Subject who uses health foods, supplements or a medicine which affects cognitive function.
(11) Subject who has undergone surgery of the gastrointestinal tract (excluding appendicitis).
(12) Subject who has participated in another clinical trial (study) within three months prior to the day of consent or subject who plans to participate in another clinical trial (study) during the trial period.
(13) Subject who has provided a blood sample or donated blood components of more than 200 mL within a month prior to the day of consent or of more than 400 mL within three months.
(14) Subject who is currently pregnant or lactating or may get pregnant or be lactating during the trial period.
(15) Subject who has difficulty in record compliance with various survey forms.
(16) Subject who is judged to be inappropriate as a participant from the clinical examination values and the measurement values of the screening.
(17) Any other subject who is judged to be inappropriate as a participant by the principal investigator.

Those with suspected dementia were excluded by setting the MMSE score at 22 or more. Regarding the MMSE score, a cutoff value of 23 is generally used to determine that those having the value or less are suspected to have dementia, but the specificity was increased by setting the cutoff value at 22 or less.

Additionally, 80 individuals with suspected mild cognitive impairment (MCI) were chosen by mainly choosing those having a lower RBANS score.

After conducting the Japanese-version of the MCI Screen, the 80 chosen individuals were divided equally into two groups of the probiotic group (*Bifidobacterium breve* FERM BP-11175 consumption group) and the placebo group through stratified randomization using the gender, the age and the RBANS score as the assignment factors. As the test foods, two kinds of capsule food shown in Table 1 were produced. The participants in the probiotic group have taken two probiotic capsules (containing 10 billion cells or more of *Bifidobacterium breve* FERM BP-11175 per capsule) per day for 16 weeks, and the participants in the placebo group have taken two placebo capsules (containing starch in the capsule) per day for 16 weeks. After 16 weeks, the RBANS and the Japanese-version of the MCI Screen were conducted again, and the scores before and after consumption were compared.

### [Table 1]

**Table 1**

| Test Food Name | Probiotic | Placebo |
|---|---|---|
| Relevant Component | *Bifidobacterium breve* FERM BP-11175 (10 billion cells (1.0×10¹⁰ cells) or more as living bacterium) | - |
| Additives | - | Starch |
| | Milk Protein Digest | Milk Protein Digest |
| | HPMC | HPMC |
| | Na Alginate | Na Alginate |
| | Ca Carbonate | Ca Carbonate |
| | Ca Phosphate | Ca Phosphate |
| | Colorant | Colorant |
| Expiration Date | 2 Years after Production Date | |
| Storage Method | Room Temperature | |

| | | |
|---|---|---|
| ∗1 Per capsule | | |

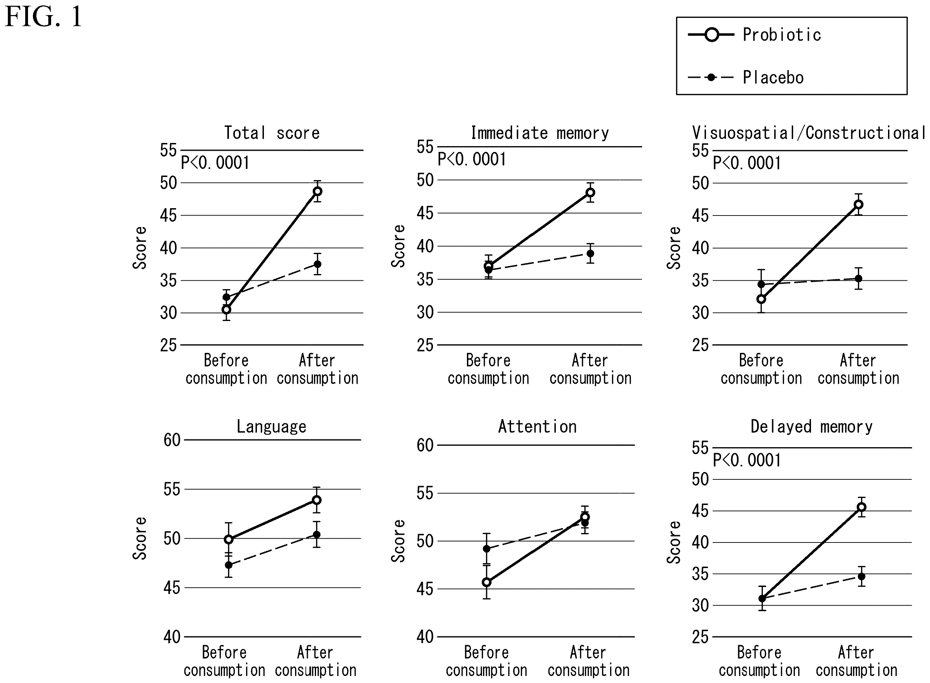

From the comparison of the actual values of the total assessment scores and the five cognition domains below in the RBANS of the *Bifidobacterium breve* FERM BP-11175 consumption group and the placebo group between before and after consumption, improvement of the total assessment score and the domains of immediate memory, visuospatial/constructional and delayed memory was observed in the *Bifidobacterium breve* FERM BP-11175 consumption group (Fig. 1).

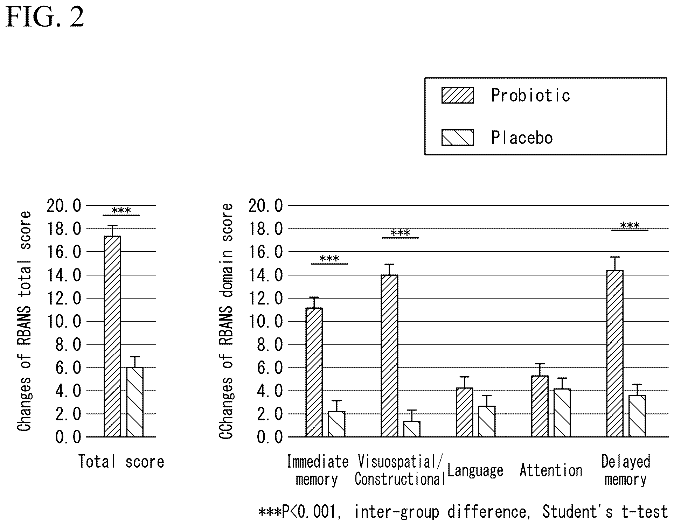

Regarding the changes of RBANS domain score, the total assessment score and the domains of immediate memory, visuospatial/constructional and delayed memory also improved in the *Bifidobacterium breve* FERM BP-11175 consumption group (Fig. 2) .

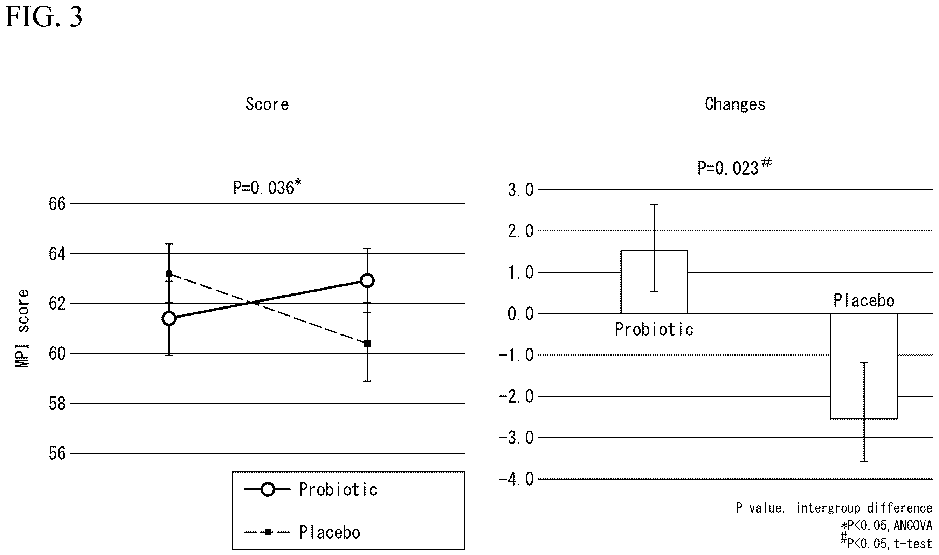

Also in the Japanese-version of the MCI Screen, improvement of the comparison before and after consumption and the changes was similarly observed in the *Bifidobacterium breve* FERM BP-11175 consumption group (Fig. 3).

From the results of the RBANS and the Japanese-version of the MCI Screen, *Bifidobacterium breve* FERM BP-11175 showed an effect of maintaining·improving cognitive function, which declines with the age.

In this regard, the functions of the domains of immediate memory, visuospatial/constructional and delayed memory, which improved in the RBANS, are all known to be governed by the hippocampus. Therefore, it is suggested that *Bifidobacterium breve* FERM BP-11175 has improved hippocampus function.

The Japanese-version of the MCI Screen (JMCIS) is an examination which can quantitatively express a minute change in cognitive function in the healthy to border to MCI regions easily with high accuracy by interactive checking for around 10 minutes. Based on the information on the subject, such as the gender, the age, the learning period (years) and the race, the memory performance index is calculated using the unique algorithm and database (provided by MILLENNIA Corporation).

## Claims

1. A cognitive function-improving agent containing one or both of *Bifidobacterium breve* FERM BP-11175 and a culture of *Bifidobacterium breve* FERM BP-11175 as an active ingredient for administration to a subject who has not developed dementia.

2. The cognitive function-improving agent according to claim 1, wherein the subject who has not developed dementia is a subject who has not developed dementia but who is concerned about age-related decline in cognitive function of the brain.

3. The cognitive function-improving agent according to claim 1, wherein the subject who has not developed dementia is a subject who has not developed dementia but who is concerned about age-related decline in combined cognitive function.

4. The cognitive function-improving agent according to claim 1, wherein the subject who has not developed dementia is selected from the group consisting of healthy individuals, the group consisting of individuals with mild cognitive impairment and the group consisting of healthy individuals and individuals with mild cognitive impairment.

5. The cognitive function-improving agent according to claim 1, wherein the subject who has not developed dementia is a subject having an MMSE score of 22 or more and having a total RBANS score of 65 or less or a Japanese-version of the MCI Screen score of 75 or less.

6. The cognitive function-improving agent according to any one of claims 1 to 5 which contains 1×10⁶ to 1×10¹² CFU/g of *Bifidobacterium breve.*

7. A cognitive function-improving food or drink containing one or both of *Bifidobacterium breve* FERM BP-11175 and a culture of *Bifidobacterium breve* FERM BP-11175 for a subject who has not developed dementia.

8. Use of one or both of *Bifidobacterium breve* FERM BP-11175 and a culture of *Bifidobacterium breve* FERM BP-11175 for the manufacture of a cognitive function-improving agent for administration to a subject who has not developed dementia.

9. A cognitive function-maintaining agent containing one or both of *Bifidobacterium breve* FERM BP-11175 and a culture of *Bifidobacterium breve* FERM BP-11175 as an active ingredient for administration to a subject who has not developed dementia.

10. The cognitive function-maintaining agent according to claim 9, wherein the subject who has not developed dementia is a subject who has not developed dementia but who is concerned about age-related decline in cognitive function of the brain.

11. The cognitive function-maintaining agent according to claim 9, wherein the subject who has not developed dementia is a subject who has not developed dementia but who is concerned about age-related decline in combined cognitive function.

12. The cognitive function-maintaining agent according to claim 9, wherein the subject who has not developed dementia is selected from the group consisting of healthy individuals, the group consisting of individuals with mild cognitive impairment and the group consisting of healthy individuals and individuals with mild cognitive impairment.

13. The cognitive function-maintaining agent according to claim 9, wherein the subject who has not developed dementia is a subject having an MMSE score of 22 or more and having a total RBANS score of 65 or less or a Japanese-version of the MCI Screen score of 75 or less.

14. The cognitive function-maintaining agent according to any one of claims 9 to 13 which contains 1×10⁶ to 1×10¹² CFU/g of *Bifidobacterium breve.*

15. A cognitive function-maintaining food or drink containing one or both of *Bifidobacterium breve* FERM BP-11175 and a culture of *Bifidobacterium breve* FERM BP-11175 for a subject who has not developed dementia.

16. Use of one or both of *Bifidobacterium breve* FERM BP-11175 and a culture of *Bifidobacterium breve* FERM BP-11175 for the manufacture of a cognitive function-maintaining agent for administration to a subject who has not developed dementia.

17. A hippocampus function-improving agent containing one or both of *Bifidobacterium breve* FERM BP-11175 and a culture of *Bifidobacterium breve* FERM BP-11175 as an active ingredient.

18. The hippocampus function-improving agent according to claim 17 for administration to a subject who has not developed dementia.

19. The hippocampus function-improving agent according to claim 18, wherein the subject who has not developed dementia is selected from the group consisting of healthy individuals, the group consisting of individuals with mild cognitive impairment and the group consisting of healthy individuals and individuals with mild cognitive impairment.

20. The hippocampus function-improving agent according to claim 18, wherein the subject who has not developed dementia is a subject having an MMSE score of 22 or more and having a total RBANS score of 65 or less or a Japanese-version of the MCI Screen score of 75 or less.

21. The hippocampus function-improving agent according to any one of claims 17 to 20 which contains 1×10⁶ to 1×10¹² CFU/g of *Bifidobacterium breve.*

22. A hippocampus function-improving food or drink containing one or both of *Bifidobacterium breve* FERM BP-11175 and a culture of *Bifidobacterium breve* FERM BP-11175.

23. Use of one or both of *Bifidobacterium breve* FERM BP-11175 and a culture of *Bifidobacterium breve* FERM BP-11175 for the manufacture of a hippocampus function-improving agent.

24. A hippocampus function-maintaining agent containing one or both of *Bifidobacterium breve* FERM BP-11175 and a culture of *Bifidobacterium breve* FERM BP-11175 as an active ingredient.

25. The hippocampus function-maintaining agent according to claim 24 for administration to a subject who has not developed dementia.

26. The hippocampus function-maintaining agent according to claim 25, wherein the subject who has not developed dementia is selected from the group consisting of healthy individuals, the group consisting of individuals with mild cognitive impairment and the group consisting of healthy individuals and individuals with mild cognitive impairment.

27. The hippocampus function-maintaining agent according to claim 25, wherein the subject who has not developed dementia is a subject having an MMSE score of 22 or more and having a total RBANS score of 65 or less or a Japanese-version of the MCI Screen score of 75 or less.

28. The hippocampus function-maintaining agent according to any one of claims 24 to 27 which contains 1×10⁶ to 1×10¹² CFU/g of *Bifidobacterium breve.*

29. A hippocampus function-maintaining food or drink containing one or both of *Bifidobacterium breve* FERM BP-11175 and a culture of *Bifidobacterium breve* FERM BP-11175.

30. Use of one or both of *Bifidobacterium breve* FERM BP-11175 and a culture of *Bifidobacterium breve* FERM BP-11175 for the manufacture of a hippocampus function-maintaining agent.
